# FASCICULE DE BREVET EUROPEEN

(11) **EP 3 057 981 B1**
(45) Date de publication et mention de la délivrance du brevet: **10.11.2021**
(21) Numéro de dépôt: 14784247.0
(22) Date de dépôt: 15.10.2014
(51) Int. Cl.: A61K 35/13, A61K 39/00, A61P 35/00, C07K 14/47, A61K 38/17, A61K 39/385

(54) **VACCIN THÉRAPEUTIQUE CONTRE LE CANCER À BASE DE PROTÉINES DE STRESS RENDUES IMMUNOGÈNES**
THERAPEUTISCHER KREBSIMPFSTOFF AUF BASIS VON IMMUNOGENEN STRESSPROTEINEN
THERAPEUTIC CANCER VACCINE BASED ON STRESS PROTEINS RENDERED IMMUNOGENIC

(30) Priorité: 15.10.2013 FR 1360031
(43) Date de publication de la demande: 24.08.2016
(73) Titulaire: BRENUS PHARMA, 63500 Issoire (FR)
(72) Inventeur: PINTEUR, Benoît, F-69210 Lentilly (FR); DEVILLERS, Gilles, F-69210 Lentilly (FR)
(74) Mandataire: Icosa
(86) Numéro de dépôt international: PCT/EP2014/072149
(87) Numéro de publication internationale: WO 2015/055733

(56) Documents cités:
- WO-A1-01/14411
- WO-A1-97/06821
- WO-A1-99/07860
- WO-A2-00/38710
- ANAS YOUNES: "A phase II study of heat shock protein-peptide complex-96 vaccine therapy in patients with indolent non-Hodgkin's lymphoma", CLINICAL LYMPHOMA, vol. 4, no. 3, 1 décembre 2003 (2003-12-01), pages 183-185, XP055123459,
- CAUDILL M M ET AL: "HSPPC-96: a personalised cancer vaccine.", EXPERT OPINION ON BIOLOGICAL THERAPY MAY 2001, vol. 1, no. 3, mai 2001 (2001-05), pages 539-547, XP009178564, ISSN: 1471-2598
- A. MÉNORET ET AL: "Protéines de choc thermique et antigènes tumoraux", MÉDECINE/SCIENCES, vol. 10, no. 6-7, 1 janvier 1994 (1994-01-01), pages 665-671, XP055123188, ISSN: 0767-0974, DOI: 10.4267/10608/2683
- None

## Description

La présente invention est relative à une nouvelle approche thérapeutique du traitement du cancer basée sur les protéines de stress « protéines chaperon » de type HSP et/ou GRP ou autres protéines impliquées dans les mécanismes de résistance (MPRs, LRPs, CTL4, DP-L1, etc.). Elle concerne notamment un procédé de préparation de compositions pharmaceutiques ou de vaccins thérapeutiques, et l'utilisation de celles-ci dans une méthode de traitement thérapeutique du cancer.

### ÉTAT DE LA TECHNIQUE

Le système immunitaire repose sur deux mécanismes de défense : l'immunité innée, rapide mais non spécifique et l'immunité acquise, plus lente, mais spécifique et dotée d'une mémoire. Ces deux mécanismes complémentaires permettent de lutter contre les « agressions » internes en mobilisant soit directement les cellules lors de la réponse immunitaire à médiation cellulaire, soit par la sécrétion de molécules actives (type immunoglobulines, cytokines, etc.) lors de la réponse immunitaire à médiation humorale.

L'immunité intervient très largement dans la survenue des cancers. D'après la théorie des trois phases (3E), le processus de cancérisation repose sur une double fonction immunitaire, l'immunosurveillance protectrice visant à détruire les cellules tumorales et la « sélection » de cellules cancéreuses résistantes, en réponse au mécanisme d'élimination. La théorie des trois phases comprend la première phase dite d'élimination, au cours de laquelle le système immunitaire lutte contre la prolifération tumorale en faisant intervenir des modifications tissulaires et environnementales liées à la tumeur (mobilisation de cellules non spécifiques (macrophage, NK, DC)), sécrétion de molécules anti-prolifératives, apoptotiques, angiostatiques, production de cytokines, mobilisation et activation des CD4 et CD8. Lors de la deuxième phase, dite d'équilibre, en réponse au système immunitaire, les cellules tumorales « sensibles » sont éliminées et une sélection de cellules les plus résistantes s'opère. Les mécanismes de résistance mis en œuvre sont une résistance à l'apoptose, la sécrétion de cytokines inhibitrices (TGF-β, IL10, PGE2, IDO), l'altération de la présentation d'antigène (perte partielle ou complète de l'expression du CMH de classe I), la sécrétion de molécules neutralisantes et expression MICA et MICB, une « contre-attaque » du système immunitaire à médiation cellulaire par expression de Fas-L, PD-L1 entraînant la mort des lymphocytes T. Lors de cette phase, le nombre de cellules tumorales détruites est en équilibre avec le nombre de cellules résistantes. Cette phase d'équilibre correspond à la phase de rémission observée dans les traitements, c'est aussi une phase de sélection des clones les plus virulents par leur capacité de résistance au système de défense immunitaire ou au traitement. Lors de la troisième phase, dite d'échappement, les cellules résistantes aux différents mécanismes de protection du système immunitaire ayant développé un ou plusieurs moyens d'échappement prolifèrent en absence de tout contrôle. Les cellules cancéreuses développent alors une masse tumorale qui est la manifestation clinique du phénomène d'échappement physiologique. Un phénomène d'échappement apparenté est aussi observé dans les stades avancés et métastasiques des cancers lors des phénomènes de résistance aux traitements.

Les traitements anticancéreux comprennent la chirurgie, dont l'objectif est principalement de retirer ou de réduire la tumeur, mais cette action « mécanique » n'a pas de réel effet inhibiteur sur le processus de cancérisation et est généralement complétée par différents traitements visant à « éliminer » l'origine du cancer. La radiothérapie vise à provoquer une altération de l'ADN des cellules à prolifération rapide, ce qui est le cas des cellules tumorales. Les effets secondaires de la radiothérapie sont doubles : les cellules saines sont-elles même irradiées, provoquant leur destruction ou des modifications génétiques pouvant entraîner leur « cancérisation », et les cellules tumorales développent des résistances à l'apoptose radio-induite par production de protéines Chaperon ou chaperonnes (HSP, GRP, etc.) entraînant un phénomène d'échappement. La chimiothérapie vise à éliminer les cellules tumorales en agissant soit sur les cellules elles-mêmes, soit en inhibant des voies métaboliques spécifiques : interaction directe avec l'ADN (agents électrophiles, agents intercalants, agents scindants), interaction indirecte avec l'ADN (inhibiteurs de la synthèse d'ADN tels que antimétabolites, inhibiteurs de la topoisomérase ; inhibiteur de la formation du fuseau), inhibiteurs de la néovascularisation, inhibiteurs du protéasome. Là aussi, des mécanismes de résistance sont développés par les cellules tumorales et malgré les stratégies de poly-chimiothérapies mises en œuvre, on observe des phénomènes de rechute avec des proliférations massives, preuve d'une adaptabilité et d'une sélection des cellules que l'on peut rapprocher des phénomènes d'échappement naturels tels que décrits dans la théorie des 3E impliquant le système immunitaire.

L'immunothérapie comprend l'immunothérapie passive qui consiste à fournir une quantité importante d'effecteurs et l'immunothérapie active qui a pour but d'induire une réponse immunitaire spécifique. L'Immunothérapie passive est basée sur l'injection d'anticorps pour bloquer un récepteur, induire une lyse cellulaire, stimuler la cytotoxicité, lever l'inhibition à l'apoptose (cetuximab-Erbitux®, Bevacizumab-Avastin®, Rituximab-Mabthera®, etc.). L'utilisation de cytokines constitue une autre stratégie pour stimuler des réponses immunitaires protectrices contre la tumeur (IL-2, INFγ, etc.). L'immunothérapie active ou immunisation par vaccination a pour but d'immuniser les patients contre le cancer selon différentes stratégies : activation du système immunitaire par activation de cellules dendritiques, agoniste de TLR, lysat de cellules tumorales, cellules tumorales non prolifératives modifiées ou pas. Ces approches constituent les nouvelles thérapies innovantes (ATMP). Elles sont prometteuses et donnent de bons résultats d'autant plus si elles sont couplées avec d'autres traitements. Le premier traitement d'immunothérapie active ayant été approuvé par la FDA est le Sipuleucel-T Provenge® basé sur l'activation des LT du patient par culture en présence d'antigènes tumoraux spécifiques. Cependant on observe aussi avec ces traitements des phénomènes d'échappement similaires aux phénomènes d'échappement naturels tels que décrits dans la théorie des 3E impliquant le système immunitaire.

Il existe toujours un besoin de proposer de nouvelles stratégies thérapeutiques pour le traitement du cancer, qui puisse apporter une efficacité propre et/ou contribuer à une stratégie multi-thérapeutique.

Les différentes mécanismes anti-tumoraux, bien que multiples, ont tous pour stratégie l'attaque directe ou indirecte des cellules tumorales avec, comme ultime étape, la lyse de la cellule ou son apoptose. Le facteur de résistance à l'apoptose semble donc être un phénomène prépondérant car il induit un échappement direct (inhibition de l'apoptose induite) ou indirecte en protégeant les cellules tumorales contre des mécanismes intermédiaires de destruction.

De nombreuses études ont permis de mieux comprendre les phénomènes de résistance et en particulier le rôle des « protéines chaperon » ou protéines de stress, dans la résistance à l'apoptose. Il est notamment connu l'effet protecteur des Heat Schock Proteins (HSP 110, HSP 90, HSP 70, HSP 60, HSP 20 et l'ubiquitine) et des GRP (glucose regulated proteins) GRP94, GRP78 (BiP) et GRP58, localisées principalement dans le réticulum endoplasmique (aussi classées dans la famille des protéines de stress). Il est aussi mis en évidence des facteurs de résistance liés plus spécifiquement aux traitements (Multi Drug Resistance) dont les Multidrug Résistance Proteins (MRP) telles que la GP 170, la VMP1 et les LRP.

Le mécanisme de résistance au stress et notamment au choc thermique est semble-t-il universel et présent dans tous les organismes vivants (bactéries, plantes, animaux). La fonction des HSP est caractérisée par l'activité protectrice et réparatrice de certaines protéines et enzymes en formant des complexes moléculaires inhibant la dénaturation et la formation de « liaisons impropres » suite à des agressions « métaboliques » (hypoxie, faible concentration glucidique, etc.), physiques (thermique, radiation) ou chimique/médicamenteuse.

Il existe aujourd'hui de nouvelles stratégies utilisant les HSP comme cible thérapeutique. Des molécules ont notamment été développées pour tenter d'inhiber les HSP 90, avec cependant des résultats contrastés et une toxicité élevée. L'une des thérapies les plus avancée est l'utilisation de la petite molécule 17-AAG (Tanespimycin ou 17-allylamino-17-demethoxygeldanamycin ; Neckers, 2007. J Biosci. 32(3):517-30), qui inhibe la fonction chaperonne de HSP 90 et dont l'activité clinique est prometteuse mais avec de nombreux effets secondaires. De même, la petite molécule Ganetespid (STA-9090), un inhibiteur du site ATP de l'HSP 90, est actuellement testée dans le mélanome, comme le sont aussi d'autres molécules agissant sur des fonctions ou des sites de l'HSP 90 (Novobiocine, Shepherdine, inhibiteurs des isoformes, etc.). Une autre cible à l'étude est la levée de l'inhibition de l'apoptose en agissant sur l'HSP 70. Les produits à l'étude sont ADD70 (molécules d'acides aminés), PES (2-phényléthylenesulfonamide), VER-155008 (dérivé d'adénosine), MKT077 (petite molécule). Enfin, l'utilisation de « anti-sens OGX-427 » dirigé contre l'HSP 27 et la Clusterin a fait l'objet de plusieurs études cliniques.

Par ailleurs il est fait référence à l'utilisation autologue d'HSP 70 ou dérivés, comme facteur d'immunisation contre des cellules tumorales chez la souris. Ménoret & Le Pendu (**1994.** *Med Sci (Paris).* **10(6-7)**:665-71) décrivent l'immunisation de souris contre des préparations de HSP 70 purifiées qui se révèlent immunogènes à condition d'être associées à des peptides tumoraux. Wirth et al. (2002. Ann Med Vet. 146(4): 201-216) mentionnent des travaux ayant montré que la vaccination de souris avec une préparation d'HSP ou le transfert de gènes HSP 70 dérivés de tumeur, les immunise vis-à-vis d'une administration de cellules tumorales autologues et induit une régression de tumeurs déjà développées.

Cependant, à l'heure actuelle il semble que ces approches ont une visée spécifique de l'une ou l'autre des protéines de stress sans permettre une stratégie combinée visant l'inhibition de « l'ensemble » des mécanismes de protection développés par les cellules tumorales. Ainsi il a déjà été évoqué que l'inhibition de l'HSP 90 entraînerait aussi un signal de défense par stimulation de la sécrétion d'autres HSP (Heat Shock Response) comme l'HSP 27 et HSP 70, renforçant ainsi la résistance à l'apoptose, et en induisant des chimio-protections (Ménoret & Le Pendu, 1994. Med Sci (Paris). 10(6-7):665-71).

La présente invention a pour objectif de proposer une approche globale, c'est-à-dire permettant d'agir en même temps sur de multiples mécanismes de résistance, de préférence l'ensemble de ces mécanismes, qui sont mis en jeu par les cellules tumorales.

L'invention a aussi pour objectif de faire en sorte que cette approche puisse être adaptée à un stress ou au(x) stress subi(s) par les cellules tumorales *in vivo,* notamment dans le cours du protocole thérapeutique appliqué à un patient.

L'invention a aussi pour objectif de proposer une approche permettant une standardisation liée à l'emploi de lignées cellulaires.

L'invention a donc aussi pour objectif que cette approche tienne compte du type et/ou du grade des cellules tumorales, notamment celles du patient traité.

L'invention a aussi pour objectif de proposer une telle approche qui puisse à elle seule constituer un protocole thérapeutique, ou qui soit un protocole adjuvant d'un autre protocole thérapeutique.

L'invention a encore pour objectif de proposer une telle approche qui soit patient-spécifique.

### RÉSUMÉ DE L'INVENTION

L'objectif de la présente invention est de stimuler le système immunitaire par des protéines de stress rendues immunogènes, notamment par hapténisation, afin de provoquer une élimination des cellules exprimant ces facteurs de résistance (protéines de stress). Ce traitement immunologique vise à éliminer ou à contrôler les cellules pouvant exprimer ces facteurs de résistance. L'approche thérapeutique proposée vise notamment à être utilisée contre le cancer sur la base des points suivants :
- les cellules tumorales développent des mécanismes de résistance ou d'échappement quand elles sont « stressées » naturellement (système immunitaire) ou par l'intermédiaire d'un protocole thérapeutique (irradiation, chimiothérapie, immunothérapie, etc.),
- les mécanismes de résistance font intervenir les protéines de stress de type HSP, GRP, et autres protéines/glycoprotéines,
- les protéines de stress ne sont pas naturellement immunogènes par elles-mêmes *in vivo,* bien que pouvant dans certaines conditions engendrer une « immunoprotection » par vaccination,
- les approches anti-HSP (ou anti-facteurs de résistances) spécifiques semblent subir aussi un phénomène d'échappement,
- l'environnement protéique (co-facteur, substrat, etc.) semble être un élément à prendre en considération pour engendrer un effet de protection anti-tumorale par vaccination.

L'invention concerne donc notamment un procédé de préparation d'une composition contenant des cellules tumorales stressées et des protéines du stress induites par l'application d'un stress *in vitro* sur une culture de cellules tumorales, tel que défini par les revendications. Plus particulièrement, ladite méthode produit de telles protéines de stress sous forme immunogène, c'est-à-dire capable d'être reconnue par le système immunitaire du patient et de conduire celui-ci à développer une réponse immunitaire à l'encontre de ces protéines et des cellules tumorales présentant de telles protéines. La méthode comporte avantageusement une étape d'inactivation des cellules tumorales pour les rendre non prolifératives. L'invention concerne aussi des compositions contenant de telles protéines, susceptibles d'être obtenues par la mise en œuvre de la méthode de production, pour leur utilisation dans des méthodes de traitement contre le cancer par administration de telles compositions, en tant que stratégie thérapeutique principale ou combinée (notamment adjuvante) à un autre protocole thérapeutique.

Le procédé de l'invention permet de préparer une composition pharmaceutique qui comprend plusieurs ou l'ensemble ou la majorité des protéines de stress exprimées par les cellules tumorales dans les conditions du stress appliqué sur elles. Ceci constitue un avantage du produit obtenu qui, en étant représentatif d'une pluralité de protéines de stress, permet à la composition pharmaceutique de comprendre tout un ensemble de protéines de stress rendues immunogènes constituant autant d'éléments immunogènes contre lesquels le système immunitaire du patient va pouvoir réagir, puis cibler tout un panel de cellules tumorales et contrecarrer les mécanismes de résistance diversement développés par ces cellules tumorales sous la pression du système immunitaire et/ou des traitements appliqués.

Généralement, ces protéines du stress se trouveront dans les compositions susceptibles d'être obtenues par la mise en œuvre du procédé selon l'invention sous une ou plusieurs des formes suivantes : à l'état libre, à l'état associé à la membrane d'une cellule tumorale (y compris intra-membranaire), présenté à la surface d'une cellule tumorale, présente à l'intérieur d'une cellule tumorale, associée à un fragment de cellule tumorale ou à un peptide tumoral.

La composition obtenue selon le procédé de l'invention comprendra donc de préférence une ou plusieurs des protéines de stress répertoriées à ce jour et à un moment donné. La partie qui suit en présente plusieurs familles connues de l'homme du métier.

Les HSPs sont classées en six familles en fonction de leur masse moléculaire, mais leurs conformations et leurs caractéristiques au sein d'une même famille semblent variables en fonction de leur origine. Ainsi, parmi les HSP 70 (qui comportent entre autres des HSP 72 et 73), on peut faire la distinction entre celles qui répondent au choc thermique (HSP 70) et celles qui sont peu affectées par le stress thermique (HSC 70).

Les HSP 25/HSP 27, faiblement exprimées dans les cellules normales, sont surexprimées dans les cellules tumorales, et joueraient un rôle dans la résistance à certains traitements et favoriseraient la croissance et le développement prolifératif des cellules tumorales. Un autre rôle serait l'inhibition de l'apoptose par interaction avec la voie impliquant la Caspase-3, la régulation dans la production de TNF-α, etc.

La famille des HSP 47 est la plus récemment identifiée, au rôle encore peu défini. Elle participerait principalement à l'élaboration des tissus conjonctifs, notamment lors de la production de collagène. Faisant partie des « Heat Shock Protein », son implication dans la résistance des cellules tumorales est probable, notamment, sa participation lors de la néovascularisation des tumeurs. L'HSP 40 jouerait aussi un rôle de « co-chaperon » en association avec l'HSP 70.

Les HSP 60 (chaperonine) ont été identifiées chez la bactérie (GroEL pour Growth E. *Coli* large protein), puis retrouvées dans les plantes. Ces protéines chaperon participent à la conformation tertiaire de la structure protéique. Cette famille d'HSP jouerait un rôle dans l'immunité et notamment dans les maladies auto-immunes.

La famille des HSP 70 est l'une des familles les plus étudiées et c'est, avec l'HSP 90, celles qui sont les plus répandues. Il existerait plusieurs sous-classes qui seraient soit exprimées par les cellules à l'état « normal », soit induites directement suite à un stress. Les caractéristiques de ces HSP est leur capacité à interagir avec les autres protéines lors de leur traduction (folding), leur participation au transport membranaire, l'inhibition de l'apoptose comme protéine chaperon. L'activité chaperon de l'HSP 70 est complexe et fait intervenir plusieurs facteurs tels que l'HSP 40, Hip (Hsc Interactive Protein) Hop (Hsc-HSP 90 Organizing Protein) permettant l'activité ATPasique nécessaire à la formation des complexes « chaperon - substrat ». L'action anti-apoptose des HSP 70 est semble-t-il multiple et fait intervenir de nombreux mécanismes d'inhibition/activation sur les voies pro-Caspase 9,8,3 (inhibition) interaction avec la voie P53 (stabilisation de la protéine anti-P53), inhibition des facteurs pro-apoptotique (Bax), régularisation du cytochrome C, etc.

Les HSP 90 sont les plus exprimées constitutivement et sont présentes chez tous les eucaryotes. Elles sont elles aussi surexprimées lors de phénomène de stress et visent à la stabilisation et la renaturation des protéines. Plusieurs isoformes ont été identifiées selon leurs localisations, leurs origines et leurs fonctionnalités. Les HSP 90 après dimérisation ont la capacité d'interagir avec des co-chaperones et ainsi former des complexes protéiques permettant la mutation et la protection de molécules impliquées dans la transformation tumorale des cellules (stabilisation de protéines spécifiques) : P90 participe à la régulation de l'apoptose (inhibition de Bcl-2 et Apaf-1), participe à la prolifération cellulaire et à la métastase (hTERT, MMP2), inhibe le p53, participe à la mutation par modification puis stabilisation des protéines.

Les HSP 110 (ou 105) sont majoritairement induites suite à un stress. Elles participeraient à la protection des ribosomes qui sont particulièrement sensibles lors des chocs thermiques. Leur effet protecteur est principalement la dissociation des agrégats protéiques, leur re-solubilisation qui permet la réactivation de ces protéines. Les HSP 110 sembleraient être un sous-groupe des HSP 70 avec des domaines de liaisons identiques et donc certaines propriétés similaires.

Les GRPs (Glucose Regulated Protein) constituent l'autre grand groupe des protéines de stress. Généralement liées aux HSP, leur rôle principal dans les mécanismes de résistance est lié à la protection des structures protéiques. Ainsi la GRP78 (BiP) aurait un rôle de chaperon avec des protéines mal conformées devant être normalement détruites expliquant de ce fait la résistance des cellules cancéreuses. La GRP 75 connue sous la dénomination « mortaline » aurait une action anti-proliférative dans les cellules normales qui, suite à sa dérégulation participerait à la cancérisation des cellules entrainant alors l'effet inverse par inactivation de p53 participant à la régulation de l'apoptose. La GPR 94 participerait elle aussi à la protection des protéines incomplètement assemblées dont la conformation tridimensionnelle serait imparfaite. Liée à l'HSP 70 elle participe à la résistance à certains anti-cancéreux.

D'autres facteurs de résistances protéiques, décrits comme « Multi Drug Résistance », tels que la GP 170, les MPRs (sept différentes), les LRPs et plus récemment la VMP1 participent à l'échappement thérapeutique.

### DESCRIPTION DETAILLEE DE L'INVENTION

### 1. Le procédé de préparation

Un objet de l'invention est donc un procédé de préparation d'une composition comprenant des cellules tumorales stressées et des protéines de stress immunogènes desdites cellules tumorales, comprenant les étapes suivantes :
i) cultiver des cellules tumorales dans un milieu de culture ;
ii) soumettre les cellules tumorales en culture à l'étape i) à un stress, dans lequel le stress est choisi dans le groupe consistant en une irradiation, un choc thermique, un choc chimique, un stress métabolique et une combinaison d'au moins deux d'entre eux, dans lequel lesdites cellules en culture développent un mécanisme de résistance en réponse audit stress qui engendre la production des protéines de stress par lesdites cellules ;
iii) récupérer le produit de l'étape ii) comprenant des cellules tumorales stressées et des protéines de stress se trouvant en intracellulaire, libre extracellulaire, intramembranaire, et/ou en surface d'une cellule tumorale ;
iv) traiter le produit obtenu à l'étape iii) par une molécule ou un procédé capable de rendre les protéines de stress immunogènes, de préférence une hapténisation
dans lequel l'intervalle de temps entre la fin de l'étape ii) et le début de l'étape suivante est de plusieurs heures.

Le milieu de culture peut être un milieu permettant de conserver la viabilité des cellules et/ou leur croissance ou multiplication. Les milieux bien connus de l'homme du métier peuvent être utilisés. Ces milieux comportent en général un milieu de culture de base « Milieu Essentiel Minimum » permettant la survie cellulaire dans lequel on peut ajouter un ou des facteurs mitogènes par le biais de sérum ou en utilisant des facteurs de croissance définis (milieu « synthétique sans sérum »). On citera à titre d'exemple les milieux classiques suivants : DMEM, EMEM, HBSS, EBSS, PBS, RPMI, ainsi que d'autres utilisés pour la culture cellulaire sans sérum PANSERIN, Ex-cell® médium, etc., complémentés ou pas en Glutamine, Insuline, HAM's nutriment, etc., auxquels on peut, le cas échéant, ajouter du sérum et/ou des facteurs de croissance.

L'étape ii) est de préférence appliquée à un milieu de culture comprenant des cellules tumorales en phase de croissance ou en phase plateau. L'homme du métier peut aisément déterminer que les cellules se trouvent dans l'une de ces phases. En premier lieu, il est possible de déterminer à l'avance les conditions de culture permettant d'atteindre ces phases au bout d'une durée déterminée. Il est également possible de déterminer que ces phases sont atteintes par les méthodes suivantes, courbe de croissance cellulaire, viabilité, temps de doublement, dosage de métabolite ou consommation de nutriments, etc.

Selon le procédé de l'invention, le stress est appliqué *in vitro.* Il est de nature chimique ou physique, et la durée d'application du stress peut varier dans des proportions qui dépendent du type de stress appliqué, la nature et la durée de chaque stress étant choisies pour que les cellules tumorales produisent des protéines de stress dans une mesure adaptée à l'invention, par exemple pour tenir compte du protocole anti-cancéreux appliqué au patient. Selon le procédé de l'invention, les méthodes de stress suivantes sont employées :
- irradiation, notamment irradiation comprise entre environ 0,25 et environ 25 gray, de préférence faible dose, c'est-à-dire entre environ 1 et environ 5 gray, par exemple environ 2 gray ; la durée d'irradiation peut être comprise entre environ 1 et environ 20 min, de préférence entre environ 1 et environ 5 min ;
- choc thermique, par exemple application progressive ou brusque d'une température supérieure à 37 °C et suffisante pour induire un stress permettant de produire des protéines de stress thermique ; cette température peut se situer entre environ 38 et environ 45 °C, de préférence entre environ 40 et environ 43 °C ; la durée de maintien à cette température peut être comprise entre environ 20 et environ 100 minutes, de préférence entre environ 30 et environ 60 minutes ; la montée en température peut durer de environ 1 à environ 5 minutes, par exemple environ 3 minutes;
- choc chimique avec une substance permettant d'induire un stress permettant de produire des protéines de stress chimique ; parmi les substances utilisables, on peut citer les alcools, tels que l'éthanol (généralement utilisé entre environ 10% et environ 50% v/v), les substances anti-tumorales utilisées en chimiothérapie, notamment du type agents alkylants, agents intercalants, par exemple le cyclophosphamide, la doxorubicine, agents cassant pour l'ADN, par exemple le cis platine ; dans un mode de réalisation, on emploie une ou plusieurs ou l'ensemble des substance(s) médicamenteuse(s) utilisée(s) dans le protocole de chimiothérapie standard pour le type de cancer considéré ou dans le protocole appliqué au patient ; la durée de mise en présence peut aller d'environ 1 à environ 48h, de préférence de environ 20h à environ 26h ; on peut aussi appliquer au moins deux de ces stress chimiques ;
- stress métabolique, par exemple hypoxie, pH (notamment par acidification au-delà de pH 6,5) déficience en une substance utile à la croissance ou à la survie des cellules tumorales, par exemple glucose, déficience en une substance indispensable à la croissance ou à la survie des cellules tumorales, par exemple électrolytes (équilibre sodium, potassium, calcium, etc.) ; on peut aussi appliquer au moins deux de ces stress métaboliques ; ou
- une combinaison d'au moins deux de ces types de stress.

On peut aisément déterminer les conditions pour chaque type de stress, avec comme objectif de stresser ces cellules vivantes pour obtenir la production de protéines de stress, sans induire l'apoptose ou la mort des cellules tumorales ou d'un trop grand nombre d'entre elles, comme cela est indiqué *infra.* Les méthodes de Western blot et de cytométrie FACS par exemple permettent de suivre la production de protéines de stress, par exemple HSP, particulières, en utilisant des anticorps dirigés contre ces protéines. De tels anticorps sont disponibles, comme on le verra plus loin à propos de quelques exemples d'HSP. Il est aussi possible de contrôler par des essais de routine *in vitro* (par exemple des essais effet-dose) si les conditions d'un stress sont trop drastiques, entraînant la mort des cellules tumorales.

Suivant une caractéristique, lorsque l'on applique plus d'1 stress, on préfère les appliquer successivement. De préférence, on applique le premier stress, puis on observe ensuite une phase de latence avant d'appliquer le stress suivant, ainsi de suite selon le nombre de stress. Avantageusement, on effectue une centrifugation après le premier stress, un lavage, une centrifugation, puis ajout de milieu frais, phase de latence, puis stress suivant, etc.

Suivant une caractéristique du procédé de l'invention, on peut confirmer l'expression ou la production de facteurs de résistance ou protéines de stress et même la quantifier. L'homme du métier a à sa disposition des moyens le lui permettant, tels que l'analyse par cytométrie de flux, *e.g.,* FACS, ELISA, Western blot, etc. Il peut confirmer l'expression ou la production des types de protéines de stress, de leur appartenance à telle ou telle famille, et même identifier et quantifier le type particulier.

Suivant une caractéristique du procédé de l'invention, l'étape iii) peut être suivie d'une étape iii-i) dans laquelle on traite le résultat de l'étape précédente selon une ou plusieurs des modalités suivantes : concentration des matières solides et protéiques, y compris les protéines de stress et les cellules tumorales et leurs fragments, séparation ou purification des protéines de stress ou des cellules tumorales, séparation ou purification des cellules tumorales et des protéines de stress, extraction des protéines de stress.

Selon le procédé de l'invention, les protéines de stress se répartissent en intracellulaire, libre extracellulaire, intramembranaire (cellule ou fragment de cellule), en surface d'une cellule ou d'un fragment de cellule. En général, les cellules cancéreuses stressées comprennent des protéines de stress en intracellulaire, et en surface.

Suivant le procédé de l'invention, à l'étape iv), on effectue un traitement permettant de rendre les protéines de stress immunogènes ou immunocompétentes, c'est-à-dire capables de générer *in vivo* une réponse immunitaire contre elles.

Selon le procédé de l'invention, l'intervalle de temps entre la fin de l'étape d'application du stress et le début de l'étape suivante est suffisant pour que les cellules tumorales aient produit des protéines de stress. Cet intervalle peut être déterminé en faisant varier cet intervalle de temps et en déterminant ou mesurant l'évolution de l'expression des protéines de stress. Par exemple, les méthodes de Western blot et de cytométrie, e.g., FACS, utilisant les anticorps spécifiques des protéines à suivre (voir *infra*)*,* peuvent être employées. Cet intervalle est de plusieurs heures, et il peut notamment être compris entre environ 5h et environ 24h. Cet intervalle peut être inférieur à 5 heure, il est possible de le déterminer en suivant la production de protéines de stress, en utilisant les méthodes décrites ici, Western Blot et cytométrie FACS. Il peut aussi dépasser 24 heures, mais une durée aussi longue peut être inutile, ceci pouvant aussi facilement se déterminer en utilisant les méthodes de mesures susmentionnées.

Selon l'invention, on introduit dans le produit issu de iii) ou iii-i), une molécule permettant de conférer de l'immunogénicité aux protéines de stress. Cette étape iv) *in vitro* permet par exemple de lier chimiquement ladite molécule aux protéines présentes, que celles-ci soient libres ou présentes à la surface ou à l'intérieur d'une membrane cellulaire ou d'une structure cellulaire, par exemple une cellule tumorale ou un fragment de cellule tumorale. Typiquement, la molécule, *e.g*., d'hapténisation, est une molécule qui n'est pas naturellement présente dans les cellules tumorales ou dans son environnement (« non-naturally occurring molecule »).

L'homme du métier connaît les procédures permettant de rendre les protéines immunogènes (en particulier l'hapténisation) et peut donc mettre en œuvre cette étape sans difficulté. C'est un moyen de rendre les haptènes immunogènes, en associant une molécule immunogène (porteuse ou « carrier ») et un haptène (en l'occurrence les protéines de stress) lié de façon covalente à cette molécule.

On peut utiliser toute molécule porteuse ou mélange de molécules porteuses connu. A titre d'exemples, on peut citer : dinitrophényl, 2,4-dinitrofluorobenzène (DNFB), acide sulfanilique, N-iodoacetyl-N'-(5-sulfonic-naphthyl) éthylènediamine (AED), aniline, acide p-aminobenzoique, et leurs mélanges. L'homme du métier peut se référer aux travaux de K. Landsteiner (Specificity of Serological Reactions 1945, chap. V, Harvard University Press). Les molécules « porteuses » sont capables de pénétrer la membrane des cellules et d'atteindre le cytosol. Dans l'invention, les protéines de stress libres, les intracellulaires et celles liées aux membranes cellulaires sont hapténisées.

L'étape d'hapténisation comprend l'incubation *in vitro* des cellules stressées et de la molécule porteuse. Typiquement, l'incubation peut durer d'environ 15 minutes à environ 1 heure, notamment d'environ 20 à environ 40 minutes.

Cette étape est de préférence conduite sous une agitation douce permettant de maintenir les cellules en suspension ou sous une certaine agitation.

Suivant une caractéristique du procédé de l'invention, l'étape permettant de rendre les protéines immunogène (par exemple l'hapténisation) iv) peut être suivie d'une étape iv-i) dans laquelle on traite le résultat de l'étape précédente selon une ou plusieurs des modalités suivantes : concentration des matières solides et protéiques, y compris les protéines de stress et les cellules tumorales et leurs fragments, séparation ou purification des protéines de stress ou des cellules tumorales, séparation ou purification des cellules tumorales et des protéines de stress, extraction des protéines de stress.

Suivant la nature du stress et son intensité, les cellules tumorales peuvent être plus ou moins dégradées ou fragmentées. L'objectif principal de l'invention n'est pas de dégrader ou fragmenter ces cellules, mais de les conduire à exprimer leur panoplie de protéines de stress. Cela signifie qu'une certaine proportion de cellules tumorales peut rester viable.

C'est pourquoi, suivant une autre caractéristique de l'invention, on peut prévoir une étape permettant d'inactiver les cellules viables. Cette étape dite d'inactivation est réalisée en aval de l'application du stress et après le temps suffisant pour que les cellules aient exprimé les protéines de stress.

Suivant un premier mode de réalisation, le produit issu de l'étape permettant de rendre les protéines immunogènes iv) ou de l'étape iv-i) peut être traité, notamment inactivé, (étape v)) afin que les cellules tumorales éventuellement présentes soient rendues non prolifératives.

Suivant un deuxième mode de réalisation, c'est le produit issu de l'étape de stress qui peut être traité, notamment inactivé, (étape v)) afin que les cellules tumorales éventuellement présentes soient rendues non prolifératives. Dans ce cas, l'inactivation est placée entre les étapes ii) et iv).

Tout traitement d'inactivation connu de l'homme du métier peut être employé, pourvu qu'il permette de faire perdre aux cellules tumorales leur capacité à proliférer *in vivo* lorsque la composition sera administrée au patient. Cette inactivation peut notamment être réalisée par un traitement chimique (fixation éthanolique) ou par un traitement physique, e.g., irradiation forte dose (par exemple environ 25 gray). Il est possible de déterminer les bonnes conditions d'inactivation, en effectuant des tests de culture cellulaire, afin de déterminer les conditions conduisant à une absence totale de viabilité. On peut aussi utiliser l'iodure de propidium qui permet de distinguer entre cellules vivantes et cellules mortes, comme cela est connu de l'homme du métier. On peut par exemple utiliser le test à l'iodure de propidium suivi de mise en culture, comme décrit *infra.*

Suivant une autre caractéristique de l'invention, le produit issu de l'étape iv) ou de l'étape v) est formulé, par mélange du produit rendu immunogène (matière protéique et/ou cellules et/ou fragments cellulaires hapténisés) avec un véhicule ou excipient pharmaceutiquement acceptable et éventuellement un adjuvant. De préférence, l'étape comprend le mélange avec un véhicule ou excipient pharmaceutiquement acceptable et un adjuvant.

Le procédé permet de préparer une composition pharmaceutique qui comprend plusieurs, l'ensemble ou la majorité des protéines de stress exprimées par les cellules tumorales dans les conditions du stress appliqué sur elles. Ceci constitue un premier avantage au produit obtenu qui, en étant représentatif d'une pluralité de protéines de stress, permet à la composition pharmaceutique de comprendre tout un ensemble de protéines de stress rendues immunogènes contre lesquels le système immunitaire du patient va pouvoir réagir, puis cibler tout un panel de cellules tumorales et contrecarrer les mécanismes de résistance diversement développés par ces cellules tumorales sous la pression du système immunitaire et/ou des traitements appliqués.

Afin de permettre une réponse aux différents stress et de conserver l'environnement protéique, le produit qui sera soumis au stress est de préférence composé de cellules tumorales intègres. Les cellules tumorales mises en œuvre à l'étape i) peuvent être des cellules de patient, issues de biopsies, éventuellement cultivées ou maintenues en survie, éventuellement des pools issus de différents patients, notamment des cellules allogéniques (même type cellulaire) ; des cellules du patient (cellules autologues) ; des cellules allogéniques de lignées préétablies ou produites à partir de cellules de patient, ou des mélanges de telles cellules ; des mélanges de ces différents types de cellules.

Dans un mode de réalisation particulièrement adapté, ces cellules intègres sont avantageusement des cellules de lignées, et notamment de lignées de même type que la tumeur du patient à traiter (cellules allogéniques). Ces cellules sont « stimulées » par l'un des facteurs de stress, permettant ainsi la surexpression des facteurs de résistance. Par exemple, une lignée pourra être soumise à un choc thermique, un agent chimique, une irradiation, un stress métabolique ou à plusieurs de ces stress lors de sa culture. Les lignées « stimulées » ou le produit brut ou traité issu du protocole de stress, sont ensuite rendus immunogènes afin de permettre la reconnaissance des protéines de stress par le système immunitaire. Pour avoir la meilleure représentativité des facteurs de résistance, plusieurs lignées, notamment allogéniques, peuvent être utilisées.

Dans un mode de réalisation, à partir d'une même population de cellules tumorales, on soumet des groupes de cellules à des stress différents. On peut par exemple soumettre une première partie des cellules à un type de protocole de stress choisi parmi ceux décrits plus haut, puis soumettre une deuxième partie des cellules à l'un des autres protocoles de stress (nature de stress différente ou même nature de stress mais conditions différentes), etc. On peut alors les utiliser sous forme de kit-of-part pour une administration simultanée, séparée ou décalée dans le temps. On peut aussi les mélanger. On dispose d'une composition pharmaceutique contenant des protéines de stress potentiellement spécifiques à chacun des mécanismes de résistance selon le type et/ou les conditions de stress appliqué (*e.g.,* irradiation, thermique, chimique, métabolique, etc.).

Dans un mode de réalisation, on applique le procédé à un mélange de cellules tumorales allogéniques, c'est-à-dire d'un même type tumoral, ou, en variante, on applique le procédé séparément à au moins deux populations de cellules tumorales, allogéniques, puis l'on mélange les produits rendus immunogènes obtenus pour constituer la composition pharmaceutique. L'invention permet donc de proposer une telle solution qui permette une approche ciblée selon le type de cellule tumorale, c'est-à-dire l'utilisation de différents types de cellules mais toutes issues d'un même type de cancer afin de potentialiser les réactions immunitaires communes contre les facteurs de résistance exprimés par l'ensemble de ces cellules d'un même type de tumeur (cellules allogéniques) ou exprimées spécifiquement par certaines cellules tumorales.

Dans un autre mode de réalisation, pour conserver l'environnement protéique spécifique de la tumeur du patient, des cellules issues de cette tumeur (cellules autologues) sont conservées dans un milieu permettant leur viabilité et/ou leur croissance, puis soumises à un (ou plusieurs) des stress cités. Les cellules autologues ainsi stimulées expriment des protéines de stress qui sont ensuite rendues immunocompétentes lors de, par exemple, l'hapténisation subséquente.

Dans ce mode de réalisation, on applique le procédé à un mélange de cellules tumorales autologues, c'est-à-dire à des cellules tumorales provenant du patient à traiter, ou, en variante, on applique le procédé séparément à au moins deux populations de cellules tumorales autologues. On peut alors les utiliser sous forme de kit-of-part pour une administration simultanée, séparée ou décalée dans le temps. On peut aussi les mélanger. L'invention permet de proposer une approche thérapeutique personnalisée, spécifique de l'individu ayant développé la tumeur afin de conserver les facteurs de résistances et l'environnement protéique propre à la tumeur du patient en utilisant des cellules autologues.

Dans un autre mode de réalisation, on combine les solutions précédentes, avec des kit-of-parts ou mélanges de cellules autologues et de cellules allogéniques, les cellules autologues et allogéniques étant de préférence allogéniques entre elles, ou avec le mélange de leurs produits rendus immunogènes suivant le procédé de l'invention.

Lorsque l'on travaille à partir de cellules tumorales de patient, on part de préférence de cellules isolées à partir d'une biopsie ou résection.

De préférence, la composition contenant des protéines de stress immunogènes et/ou, de préférence et, des cellules tumorales inactivées est congelée ou lyophilisée.

### 2. Compositions susceptibles d'être obtenues ou produites par le procédé selon l'invention

Décrite ci-dessous est une composition susceptible d'être obtenue ou produite par le procédé de l'invention. Cette composition en tant que telle n'est pas comprise dans l'invention revendiquée.

La composition se caractérise par le fait qu'elle comprend des protéines de stress rendues immunogènes conformément à l'invention, notamment hapténisées. Cette composition peut aussi se caractériser par le fait de comprendre des cellules tumorales qui sont celles ayant produit les protéines de stress en réponse au stress selon l'étape ii), notamment des cellules tumorales et/ou des débris ou fragments de ces cellules qui ont été produits par le procédé de l'invention. Cette composition peut comprendre des protéines de stress immunogènes, notamment hapténisées, libres et/ou des protéines de stress immunogènes, notamment hapténisées, présentées à la surface ou à l'intérieur des cellules tumorales ou de leurs fragments. De préférence, la composition comprend des cellules tumorales inactivées et des protéines de stress immunogènes, notamment hapténisées.

Cette composition comprend plusieurs ou l'ensemble ou la majorité des protéines de stress exprimées par les cellules tumorales *in vitro* suite à un stress appliqué sur elles, et elles sont sous une forme immunogène, notamment hapténisée. Notamment, lorsque l'on parle de « protéines de stress », on entend au moins deux protéines de stress différentes, de préférence 2, 3, 4, 5, 6, 7, 8, 9 ou 10, voire plus, protéines du stress, ceci variant du type cellulaire et du ou des stress appliqués *in vitro.*

Dans divers modes de réalisation, la composition contient une ou plusieurs protéines de choc thermique, notamment choisies parmi celles décrites *supra,* par exemple HSP 27, HSP 70 ou HSP 90 ; HSP 27, HSP 70 et HSP 90 ; HSP 27 et HSP 70 ; HSP 90 et HSP 27 ; HSP 90 et HSP 70. Dans ces compositions, cette ou ces HSP est/sont rendues immunogènes, notamment hapténisées.

Dans d'autres modes de réalisation, la composition comprend une ou plusieurs protéines de résistance pléiotropique aux médicaments, notamment protéines de résistance à la chimiothérapie (MRP, multidrug resistance-associated protéin), par exemple GP170, les MPRs (sept différentes), les LRPs et/ou la VMP1.

Dans d'autres modes de réalisation, la composition comprend des protéines de radiorésistance ou encore des protéines de résistance à un stress métabolique.

Dans d'autres modes de réalisation, la composition comprend des GRPs, par exemple la GRP78 (BiP), la GRP 75 et/ou la Gpr 94.

Conformément à l'enseignement de l'invention, la composition peut comprendre des protéines de stress de plusieurs de ces catégories.

Plus particulièrement décrite ici est une composition pharmaceutique ou un vaccin thérapeutique ou une composition immunogène comprenant des protéines de stress rendues immunogènes, de préférence hapténisées.

Ces protéines de stress peuvent être à l'état libre, à l'état associé à la membrane d'une cellule tumorale (y compris intra-membranaire), présentées à la surface d'une cellule tumorale, présentes à l'intérieur d'une cellule tumorale, et/ou associées à un fragment de cellule tumorale ou à un peptide tumoral.

Suivant une caractéristique, la composition comprend des protéines de stress rendues immunogènes, de préférence hapténisées.

Suivant une autre caractéristique, la composition comprend des cellules tumorales qui sont celles ayant produit les protéines de stress en réponse au stress selon l'étape ii), notamment des cellules tumorales inactivées. Ces cellules peuvent comprendre et comprennent avantageusement des protéines de stress intracellulaires, hapténisées ou autrement rendues immunogènes conformément à l'invention. *In vivo,* ces protéines de stress immunogènes intracellulaires seront notamment présentées au système immunitaire par les cellules présentatrices d'antigène.

Plus particulièrement, la composition contient des cellules tumorales et/ou des fragments de ces cellules, contenant et/ou portant des protéines de stress immunogènes, de préférence hapténisées.

Suivant une caractéristique, la composition contient à la fois des protéines de stress immunogènes, de préférence hapténisées, libres et des cellules tumorales et/ou des fragments de ces cellules, contenant et/ou portant des protéines de stress immunogènes, de préférence hapténisées.

Pour une utilisation en tant que produit administrable à un patient, ces cellules sont rendues incapables de proliférer, on parlera d'inactivation.

Cette composition peut aussi comprendre un véhicule ou excipient pharmaceutiquement acceptable.

Dans un mode de réalisation, la composition comprend une population de protéines de stress immunogènes, de préférence hapténisées et/ou des cellules contenant de telles protéines, ayant subi un premier protocole de stress, et au moins une autre population de protéines de stress immunogènes, de préférence hapténisées et/ou des cellules contenant de telles protéines, ayant subi un autre protocole de stress.

Dans un mode de réalisation, la composition comprend une population de protéines de stress immunogènes, de préférence hapténisées et/ou des cellules contenant de telles protéines, et au moins une autre population de protéines de stress immunogènes, de préférence hapténisées et/ou des cellules contenant de telles protéines, les populations provenant de cellules tumorales initiales allogéniques et ces cellules ayant subi un protocole de stress en mélange ou séparément.

Dans un mode de réalisation, la composition comprend des protéines de stress immunogènes, de préférence hapténisées et/ou des cellules contenant de telles protéines, issues de cellules tumorales initiales autologues.

Dans un mode de réalisation, la composition comprend des protéines de stress immunogènes, de préférence hapténisées et/ou des cellules contenant de telles protéines, allogéniques et autologues.

Dans un mode de réalisation, la composition comprend des protéines de stress immunogènes, de préférence hapténisées et/ou des cellules contenant de telles protéines, allogéniques et autologues, pour une administration séparée, simultanée ou décalée dans le temps. La composition peut être constituée de plusieurs doses de protéines de stress rendues immunogènes et/ou de cellules contenant des protéines de stress allogéniques rendues immunogènes, notamment issues de différentes lignées (1, 2, 3, etc. lignées) et de plusieurs doses de protéines de stress rendues immunogènes et/ou de cellules contenant des protéines de stress autologues rendues immunogènes.

### 3. Utilisation comme médicament anticancéreux

L'invention a aussi pour objet les compositions susceptibles d'être obtenues par la mise en œuvre du procédé selon l'invention, pour leur utilisation comme médicament anticancéreux, dans laquelle le stress *in vitro* auquel sont soumises les cellules tumorales est de même nature que celui qu'un patient devant être traité va subir, de préférence le stress *in vitro* auquel sont soumises les cellules tumorales comprend une irradiation lorsque la composition est destinée à un patient devant être traité par radiothérapie, et/ou le stress *in vitro* auquel sont soumises les cellules tumorales comprend un choc chimique à l'aide d'un agent de chimiothérapie lorsque la composition est destinée à un patient devant être traité par ce même agent de chimiothérapie.

Suivant une autre caractéristique, les compositions sont utiles comme médicament anticancéreux pour administration à un patient qui va subir un traitement anticancéreux. Comme expliqué ci-dessus, ce traitement est susceptible d'engendrer un stress et donc la production de protéines de stress. Le patient en cours de traitement a déjà ou est suspecté d'avoir des cellules tumorales présentant des protéines de stress.

Suivant une caractéristique, la composition comporte en outre un adjuvant.

L'adjuvant est une substance qui agit en augmentant l'efficacité d'un vaccin. Cette substance peut agir en accélérant, en prolongeant et/ou en augmentant les réponses immunitaires spécifiques d'un immunogène lorsque cette substance est employée en combinaison avec cet immunogène spécifique. L'homme du métier peut se référer notamment à Recent advances in vaccine adjuvants, Singh M. & O'Hagan D.T. Pharmaceutical research 2002, Volume 19, issue 6, pp 715-728. Parmi les adjuvants utilisables, on peut mentionner notamment les cytokines et autres molécules d'immunomodulation (*i.e*., chémokines et facteurs de co-stimulation), des substances adjuvantes dérivées de microorganismes et de plantes ou qui sont synthétisées chimiquement. Selon leur nature, les adjuvants peuvent agir non seulement comme adjuvants d'immunostimulation mais aussi comme système de délivrance vaccinal. Ces systèmes de délivrance sont généralement particulaires et l'on peut citer notamment les émulsions, les microparticules (par exemple à base de polylactide-glycolyde PLG), les ISCOMS, les liposomes, qui permettent principalement d'orienter les immunogènes vers les cellules présentatrices d'antigène. Les adjuvants d'immunostimulation quant à eux sont principalement des molécules d'origine microbiologique, *e.g.,* lipopolysaccharide, monophosphoryl lipide A, CpG DNA, qui activent le système immunitaire. On peut aussi employer des adjuvants pour délivrance par voir mucosale, par exemple des entérotoxines bactériennes, notamment provenant *d'E. coli,* des entérotoxines thermolabiles, des mutants détoxifiés tels que K63 or R72.

Parmi les adjuvants utilisables, on peut citer, entre autres : hydroxyde d'aluminium, saponines (e.g., Quillaja saponin ou Quil A; voir Vaccine Design, The Subunit and Adjuvant Approach, 1995, édité par Michael F. Powel et Mark J. Newman, Plennum Press, NY and London, p. 210), Avridine® (Vaccine Design p.148), DDA (bromure de diméthyldioactadécyl-ammonium, Vaccine Design p.157), polyphosphazène (Vaccine Design p.204), émulsions huile-dans-l'eau, en particulier celles comprenant une huile minérale, du squalane (*e.g.,* émulsion SPT, Vaccine Design p. 147) ou du squalène (*e.g.,* MF59, Vaccine Design p.183), les émulsions eau-dans-l'huile, notamment celles comprenant une huile métabolisable (voir notamment WO9420071), une émulsion selon US 5,422,109, ou une triple émulsion, par exemple une émulsion eau-dans-l'huile-dans-l'eau.

Pharmaceutiquement acceptable fait référence à un véhicule ou excipient qui peut être utilisé pour administrer un immunogène à un patient sans risque allergique ou autre effet indésirable tout en préservant les caractéristiques du produit lors de sa conservation. Ceci inclut, par exemple, seul ou en mélange, l'eau, les solutions salines, les tampons phosphate, des composés protéiques, le dextrose, le saccharose, le glycérol, le DMSO, l'éthanol et analogues. On peut citer par exemple une solution saline à 0,9% NaCl, un tampon phosphate, un mélange à partir de solution de conservation commerciales.

Dans un mode de réalisation, la composition susceptible d'être obtenue par la mise en œuvre du procédé selon l'invention est destinée à être congelée, notamment entre -20 et -80°C. On ajoute alors à la composition au moins un agent cryoprotecteur et/ou l'on prépare la composition de manière usuelle pour qu'elle supporte la congélation sans dommage pour ses composants, notamment les cellules tumorales.

Dans un autre mode de réalisation, la composition susceptible d'être obtenue par la mise en œuvre du procédé selon l'invention est destinée à être lyophilisée (freeze-dried). On ajoute alors si besoin au moins un excipient usuel de lyophilisation.

La composition du véhicule ou excipient et/ou le choix de l'adjuvant peut varier selon la voie d'administration, comme cela est connu de l'homme du métier.

Le produit développé par l'invention peut avoir une composition variable, mais s'il est des caractéristiques qui peuvent être retenues, c'est qu'il comprend des protéines de stress induites *in vitro* par la soumission de cellules tumorales vivantes à un ou plusieurs stress contrôlé(s), que ces protéines de stress se retrouvent à une teneur élevée due à leur surexpression pas ces cellules tumorales en réponse au stress, et que généralement ces protéines de stress sont multiples, que ces protéines de stress, dans le produit final administrable au patient, sont hapténisées ou autrement traitées pour les rendre immunocompétentes (immunogènes).

Le principe de l'invention repose sur la capacité du système immunitaire à développer une réponse spécifique contre les facteurs de résistance des cellules tumorales (protéines de stress) après avoir été stimulé par la présence de protéine de stress rendues immunocompétentes (immunogènes) comme par exemple hapténisées. Cette réaction immunitaire éliminant les cellules tumorales, complète les actions initiées lors des traitements préalables (action d'adjuvant de la composition susceptible d'être obtenue par la mise en œuvre du procédé selon l'invention) ou permet l'activation de la réponse immunitaire comme traitement à titre principal.

Dans un mode de réalisation, l'utilisation comprend l'administration d'une quantité efficace d'une composition susceptible d'être obtenue par la mise en œuvre du procédé selon l'invention pour provoquer la réaction immunitaire.

La dose administrée peut par exemple comprendre de 10⁵ à 10⁷, notamment au moins 10⁶ ± 0.5 cellules par dose. Cette quantité de cellules peut être exprimée en cellules tumorales totales, en carbone d'origine tumorale (COT) ou en quantification protéique.

Le régime de dose peut comprendre une ou plusieurs administrations séparées dans le temps. Par exemple, on peut prévoir 2, 3, 4, 5, 6, 7, 8, 9 ou 10 administrations échelonnées avec un intervalle de 1 à 10 jours entre chaque administration.

Toute voie d'administration peut être employée. On peut ainsi citer les voies orale, nasale, rectale, parentérale. On préfère la voie parentérale, notamment par injection intramusculaire, intraveineuse, intrapéritonéale, sous-cutanée, intradermique. L'injection intradermique est particulièrement retenue.

Le traitement pourra être complété par l'administration d'immunostimulant(s) de tout type, permettant sa potentialisation (cytokine, facteur de croissance, immunomodulateur, adjuvant, etc.), que la composition thérapeutique soit ou pas déjà adjuvée.

L'utilisation peut comprendre la combinaison d'un traitement selon l'invention avec un protocole de traitement anti-cancéreux traditionnel : chirurgie, radiothérapie, chimiothérapie et/ou immunothérapie. Les compositions susceptibles d'être obtenues par la mise en œuvre du procédé selon l'invention sont administrées au patient avant, pendant ou après un ou plusieurs de ces protocoles. S'agissant de l'induction d'une réponse immunitaire, il est cependant avantageux d'administrer la composition susceptible d'être obtenue par la mise en œuvre du procédé selon l'invention avant ou pendant le protocole traditionnel, de préférence avant.

Selon l'invention, le patient est traité par les lignées allogéniques ayant exprimé des protéines de stress en relation avec le futur traitement traditionnel, afin de stimuler préalablement le système immunitaire pour obtenir une réponse contre les protéines de stress potentiellement exprimées *in vivo* et inhiber les résistances au traitement traditionnel. Par exemple, administration de composition susceptible d'être obtenue par la mise en œuvre du procédé selon l'invention, comprenant des protéines de stress et/ou des cellules exprimant des protéines de stress radio-induites, préalablement à un traitement de radiothérapie et/ou administration de composition susceptible d'être obtenue par la mise en œuvre du procédé selon l'invention, comprenant des protéines de stress et/ou des cellules exprimant des protéines de stress chimio-induites en utilisant préférentiellement la molécule thérapeutique anti-cancéreuse comme initiatrice du stress, préalablement à une chimiothérapie avec cette molécule.

### BRÈVE DESCRIPTION DES FIGURES

La présente invention va être maintenant décrite plus en détail à l'aide d'exemples pris à titre d'exemples non limitatifs se référant aux Figures.
La **Figure 1** est un Western Blot montrant l'induction de l'expression d'HSP 70 par un stress thermique. Pour faciliter la lecture, on a entouré les spots principaux d'étalonnage et d'HSP 70, ce dernier étant repéré par le repère numérique 1.

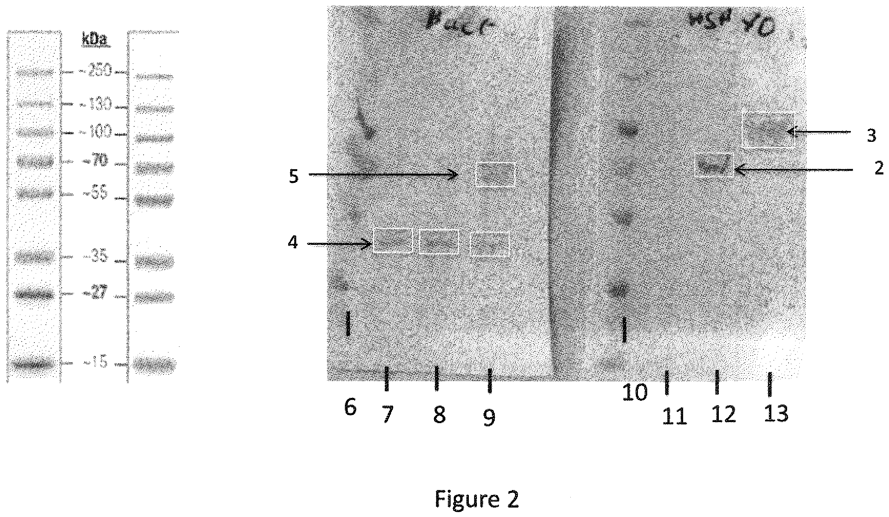

La **Figure 2** est un Western Blot montrant l'induction d'HSP 70 par stress thermique et l'hapténisation d'HSP 70, avec en parallèle un Western Blot réalisé sur un témoin β-actine. Pour faciliter la lecture, les principaux spots ont été entourés. Repères numériques : HSP 70 (2), HSP 70 hapténisée (3), β-actine (4), β-actine hapténisée (5), Marqueur poids moléculaire (6), Cellules CT26WT traitées 1 h à 37°C (7), cellules CT26WT traitées 1 heure à 42°C (8), dose vaccinale stressée, hapténisée et irradiée (9), Marqueur poids moléculaire (10), Cellules CT26WT traitées 1 h à 37°C + 14 h à 37°C (11), cellules CT26WT traitées 1 heure à 42°C + 14 h à 37°C (12), dose vaccinale stressée, hapténisée et irradiée (13).
Les **Figures 3 et 4** sont des graphes représentant chacun l'évolution dans le temps du volume de tumeurs induites chez la souris entre des souris témoins injectées avec un excipient (« Veh » pour « véhicule ») et des souris traitées avec des vaccins selon l'invention (« The » pour «Thérapeutique).
Les **Figures 5 et 6** sont des graphes montrant, pour les mêmes groupes des Figures 1 et 2, le poids de tumeur en grammes induite chez la souris au moment de la résection (« Veh » pour « véhicle » ou excipient ; « The » pour Thérapeutique).

### EXEMPLES

### Exemple 1 : Procédé de fabrication de doses allogéniques :

### Lignées cellulaires

Les lignées cellulaires sont issues de lignées préétablies commerciales (type ATCC) ou suite à la constitution de lignée issue de prélèvement de patient, caractérisées et testées. A titre d'exemple, on utilise les lignées Caov, OVCAR-3, ES-2 et OV-3, pour une cible thérapeutique cancer de l'ovaire. Elles sont mises en culture séparément dans les conditions recommandées.

### Cellules de patient

Les cellules de patient sont isolées du patient et mises en culture dans un milieu et des conditions appropriées.

Les cellules de lignées et de patient subissent au cours de leur phase de croissance ou de leur phase plateau un stress pour permettre la surexpression des facteurs de résistance (protéines de stress).

Type de stress appliqué :
- Irradiation 2 gray
- Choc thermique : montée en température jusqu'à 40°- 43°C avec un plateau de 30 à 60 minutes
- Choc chimique avec éthanol, cyclophosphamide, doxorubicin ou cis-platine.

L'expression d'un ou plusieurs facteur(s) de résistance est confirmée par cytométrie en flux, Elisa, ou Western Blot). Notamment par cytométrie en utilisant les anticorps anti-HSP (anti-human HSP27-FITC ; anti-human HSP60-PE ; anti-human HSP72-FITC ; anti-human HSP90-PE).

Les facteurs de résistance (protéines de stress) exprimés par les cellules allogéniques sont chimiquement marqués par le dinitrophelyl. En variante on peut utiliser l'acide sulfanilique, la N-iodoacetyl-N'-(5-sulfonic-naphthyl) éthylènediamine (AED), l'aniline ou l'acide p-aminobenzoique.

Les cellules allogéniques comportant les protéines de stress hapténisées sont ensuite rendues non prolifératives par irradiation forte dose (25 gray). En variante on peut utiliser la fixation à l'éthanol entre 10% et 50% V/V ou toute autre méthode permettant l'inhibition de la multiplication cellulaire tout en conservant la structure cellulaire intègre.

Les protéines de stress hapténisées cellulaires d'origine allogéniques, sont réparties sous forme de suspension cellulaire dans un milieu de formulation adapté à un usage thérapeutique et en permettant leur conservation à basse température (-20°C, -80°C), puis répartie en doses comprenant de 1 à 5.10⁶ cellules hapténisées et inactivées par dose correspondant à une dose thérapeutique exprimée en quantité de protéine « HSP positive » et/ou en quantité de matière organique. Un adjuvant d'immunisation peut être présent, par exemple BCG, GM-CSF, IL-2.

### Exemple 2 : Procédé de fabrication des doses autologues :

Les cellules sont issues de la tumeur du patient après résection. Le matériel biologique est transporté dans un kit spécifique permettant sa conservation et assurant la meilleure viabilité des cellules.

Les cellules de la biopsie sont dissociées par une méthode mécanique appropriée, puis mises en suspension dans un milieu nutritif permettant leur croissance ou leur viabilité uniquement.

Les cellules tumorales sont utilisées telles quelles ou suite à une sélection par un tri cellulaire.

Les cellules tumorales en phase d'expansion ou en phase stationnaire sont soumises à un stress tel que défini dans la fabrication des cellules allogéniques.

L'expression d'un ou plusieurs facteur(s) de résistances est confirmée selon une méthode d'analyse appropriée, comme à l'exemple 1.

Les facteurs de résistance (protéines de stress) exprimés par les cellules autologues sont chimiquement marqués par un procédé similaire à celui décrit pour les cellules allogéniques à l'exemple 1.

Les cellules autologues comportant les protéines de stress hapténisée sont rendues non proliférative par la même méthode que celle utilisée pour les cellules allogéniques à l'exemple 1.

Les protéines de stress hapténisées intégrées aux cellules autologues, sont réparties sous forme de suspension cellulaire dans un milieu de formulation adapté à un usage thérapeutique et en permettant leur conservation puis répartie en doses thérapeutiques.

### Exemple 3 : Méthode de traitement :

Le schéma thérapeutique comporte l'administration :
- d'une ou plusieurs doses allogéniques ou autologues, ou
- d'une ou plusieurs doses allogéniques et autologues, administrées conjointement ou de façon séquentielle.

L'administration du produit est une injection intradermique. On peut aussi employer une autre voie d'administration et en particulier *per os.*

Le produit est administré seul ou en synergie avec tout autre thérapie permettant une potentialisation du traitement.

### Exemple 4 : Production d'HSPs

La ligne CT26-WT est une lignée du carcinome de colon de souris, disponible sous ATCC® CRL-2638™. Les cellules poussent facilement et rapidement (temps de doublement 22 h).

La lignée HL60 est une lignée de cellules humaines (Caucasian promyelocytic leukemia) disponibles chez Sigmea-Aldrich®. Les cellules sont congelées après expansion.

Les cellules initiales ont été reçues sous forme congelée. On a décongelé les cellules, on les a mises en culture dans des flasques, en milieu de culture approprié. Le milieu de culture a été changé à J+1. Après expansion, on a effectué une numération des cellules vivantes, puis ajusté la concentration à environ 2.10⁶ cellules vivantes/mL de milieu de culture. Un pool de 200.10⁶ cellules a été récupéré, puis lavé après avoir vérifié que les cellules étaient à 50% de confluence environ. La suspension a ensuite été divisée en tubes de 50 mL à raison de 25 mL par tube. Les tubes ont été plongés dans un bain-marie chauffé à 42°C. Les tubes sont restés environ 1h dans le bain-marie. On a ensuite transféré les cellules dans des flasques à raison de 2.10⁶ cellules. Les cellules ont ensuite été incubées 14 h à 37°C. On a ensuite ajusté la concentration cellulaire à 5.10⁶ cellules vivantes/mL.

Les cellules ont ensuite été hapténisées avec une solution de 2,4-dinitrofluorobenzène (DNFB) 0,07%. L'hapténisation est révélée par un marquage au FACS. La cytométrie en flux FACS a été choisie comme technique d'analyse, elle permet de donner un pourcentage de cellules hapténisées par rapport au nombre de cellules total.

La suspension cellulaire est ajustée à une concentration de 2 à 5.10⁶ cellules/mL avec du milieu de congélation et stockée dans un congélateur à -80 ± 3°C pendant un minimum de 24 h avant de les tester.

On a ensuite soumis les cellules congelées à une irradiation par rayons-X 25 grays, afin d'inactiver les cellules tumorales.

Deux techniques standard ont été utilisées pour détecter l'expression d'HSPs ou d'HSPs hapténisées ; ces mêmes techniques peuvent être utilisées pour détecter n'importe quelle protéine de stress, moyennant les anticorps spécifiques de la protéine à détecter :
- Western blot utilisant les anticorps spécifiques de l'HSP à détecter.
- Cytométrie de flux (FACS, Fluorescence-activated cell sorting) après marquage des HSPs intracellulaires à l'aide des anticorps spécifiques.
- Comme anticorps spécifiques, on a utilisé des anticorps commerciaux anti-HSP 27, anticorps anti-HSP 70, anticorps anti-HSP 90α/β. Selon la technique utilisée, on a utilisé, comme cela est connu en soi, des anticorps complémentaires, IgG1 de souris, IgG de mouton anti-souris conjugué à de la FITC, anticorps contrôle (anti-KLH FITC) anticorps (anti-TNP FITC), spécifique du DNFB. Des anticorps commerciaux utilisables sont disponibles auprès de Santa Cruz Biotechnology Inc. et de BD Biosciences.

Pour la cytométrie, on a décongelé les cellules hapténisées, on les a lavées et on a ajusté la concentration de 1 à 2.10⁶ cellules/mL. En plaque 96 puits fond V on a déposé :
- 5 µL d'anticorps contrôle (anti-KLH FITC) dans deux puits,
- 5 µL d'anticorps (anti-TNP FITC) dans deux autres puits.

On a ensuite ajouté 100 µL de suspension de cellules hapténisées par puits, puis incubé la plaque 15 minutes à +5°C ±3°C. On a ensuite lavé les cellules, puis on a transféré les suspensions cellulaires en tubes pour passage sur un FACS Calibur. On a passé les tubes "DNP-FITC" selon les paramètres fixés au préalable avec le contrôle.

### Résultats :

### 1. Lignée CT26-WT :

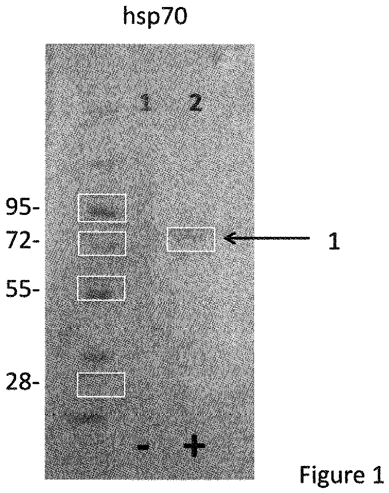

Par rapport à la même lignée maintenue à 37°C pendant 1 h, on voit apparaître en Western blot une bande à environ 70 kD (Figure 1) correspondant à HSP 70 dans les cellules stressées à 42°C selon le protocole ci-dessus.

### 2. Lignée HL60 :

L'analyse des cellules témoin (1 h à 37°C + 14 h de temps de récupération à 37°C) et des cellules stressées (1h à 42°C + 14 h de temps de récupération à 37°C) a été effectuée par cytométrie de flux après marquage intracellulaire des HSP 27, 70 et 90 par les anticorps spécifiques mentionnés *supra.*

### Résultats :

| | % cellules analysées (fenêtre) | Moyenne d'intensité des cellules marquées |
|---|---|---|
| Pas de stress | | |
| Contrôle positif | 92,07 | 59,42 |
| HSP 27 | 92,37 | 37,09 |
| HSP70 | 92,14 | 7,85 |
| HSP90 | 91,78 | 31,19 |
| Stress | | |
| Contrôle positif | 71,56 | 68,11 |
| HSP 27 | 72,07 | 71,90 |
| HSP 70 | 70,47 | 30,90 |
| HSP 90 | 70,32 | 57,03 |

On note une surexpression des HSP et plus particulièrement des HSP 27 et 70 dans les cellules stressées.

### Exemple 5 : Expérimentation animale

La fabrication des doses tumorales a consisté à la mise en expansion de cellules CT26.WT, puis à une congélation en milieu de culture supplémenté avec 5 % de DMSO et à raison de 6,25.10⁴ cellules dans 250 µL final.

La fabrication des doses vaccinales a consisté à la mise en expansion des cellules CT26.WT, puis après 2 passages les cellules ont subi un stress thermique de 1 h à +42°C suivi d'un temps de récupération de 14h à 37°C. Ensuite, les cellules ont été hapténisées (par du DNFB, dinitrofluorobenzène), puis congelées à -80°C à raison de 6,25.10⁵ cellules dans 250 µL final et enfin les doses vaccinales ont été irradiées (25 gray).

Des contrôles ont été effectués pour les doses tumorales et vaccinales :

### Doses tumorales (production de la tumeur chez l'animal):

- Stérilité par hémocultures → hémocultures négatives.
- Contrôle endotoxines : → endotoxines ≤200 UI/mL.
- Contrôle de la quantité cellulaire et viabilité des doses tumorales : → quantité comprise entre 5,5 et 6,95.10⁵ cellules, viabilité ≥ 95%.

### Doses vaccinales :

- Stérilité par hémocultures : → hémocultures négatives.
- Contrôle endotoxines : 2 → endotoxines ≤200 UI/mL.
- Contrôle de l'hapténisation par marquage FACS à l'aide de l'anticorps anti-KLH-TNP: → % d'hapténisation ≥ 96%.
- Contrôle de la quantité cellulaire des doses tumorales : → quantité comprise entre 5,96 et 6,10.10⁵ cellules), Contrôle de l'expression des HSP par cytométrie en utilisant les anticorps spécifiques mentionnés *supra* : → on a détecté l'expression des HSP 27, 70 et 90.
- Contrôle détection des HSP par Western blot en utilisant les anticorps spécifiques mentionnés *supra* : → la figure 2 montre un spot HSP 70 après stress thermique et un spot HSP 70 hapténisé.
- Test de viabilité afin de s'assurer que les cellules de la composition vaccinale ne sont pas prolifératives : ajout d'iodure de propidium + mise en culture ; l'absence de capacité de prolifération des cellules composant les doses vaccinales a été confirmée.

### Exemple 6 : Etude in vivo chez la souris.

Des souris BALB/c mâles et femelles âgées de 6 à 8 semaines, obtenues auprès de Charles River, ont été utilisées. 5 souris ont été placées par cage 16x19x35 cm sous température contrôlée (22 ± 2°C, dans des conditions d'éclairage alterné (cycles de 12 h de jour et 12 h de noir) et alimentées en eau et nourriture *ad libitum.* Les souris ont été acclimatées pendant au moins 1 semaine avant de débuter les expériences.

Le modèle de tumeur est le carcinome CT26 sous-cutané (CT26WT, ATCC® CRL-2638™), qui est un modèle de tumeur syngénique communément utilisé pour l'étude d'applications thérapeutiques contre le cancer chez l'animal, en particulier pour tester des protocoles d'immunothérapie et étudier la réponse immunitaire.

50 souris femelles ont été réparties en 5 groupes de 10. Chaque souris a été injecté par voie sous-cutanée (SC) au jour 0 (J0) avec 5.10⁴ cellules tumorales CT26WT. Le protocole de traitement a été le suivant :
- Groupe 1 : excipient
- Groupe 2 : traitement thérapeutique 1
- Groupe 3 : traitement thérapeutique 2

De la cyclophosphamide a été injectée à 15 mg/kg par voie intrapéritonéale (IP) à J+2 aux groupes 2 et 3.

Traitement 1 (BCG/IL-2/vaccin) pour le groupe 2 :
- BCG : 2.10⁶ UFC (unités formatrices de colonies) par injection SC
- IL-2 murine recombinante : 4 000 UI par injection SC
- Vaccin : 5.10⁵ cellules CT26WT (irradiées et hapténisées) par injection SC.

Traitement 2 (BCG/GM-CSF/vaccin) pour le groupe 3 :
- BCG : 2.10⁶ UFC par injection SC
- GM-CSF murin recombinant : 25 000 UI (unité internationale) par voie SC
- Vaccin : 5.10⁵ cellules CT26WT (irradiées et hapténisées) par injection SC.

### Protocoles :

| Jour | Traitement/voie | Groupe 1 | Groupe 2 | Groupe 3 |
|---|---|---|---|---|
| J 0 | CT26WT SC | X | X | X |
| J+2 | Cyclophosphamide IP | | X | X |
| J + 7 | Traitement SC | Excipient | T1 | T2 |
| J+14 | Traitement SC | Excipient | T1 | T2 |
| J+21 | Traitement SC | Excipient | T1 | T2 |
| J+28 | Traitement SC | Excipient | T1 | T2 |

Paramètres mesurés au cours de l'étude et autres spécifications :
- Contrôle journalier du comportement des souris.
- Contrôle 3 fois par semaine du poids corporel et du volume tumoral. Le volume de tumeur en mm³ a été calculé à l'aide de la formule : volume = [(largeur)² x longueur] / 2. Mesure dimensions à l'aide d'un caliper.
- Les souris sont sacrifiées en cas de signes de détresse inattendus.
- Les souris ont été sacrifié lorsque la tumeur atteint 1000 m³ et en tous cas au plus tard 40 jours après l'implantation de la tumeur. Les tumeurs ont été excisées et mesurées.

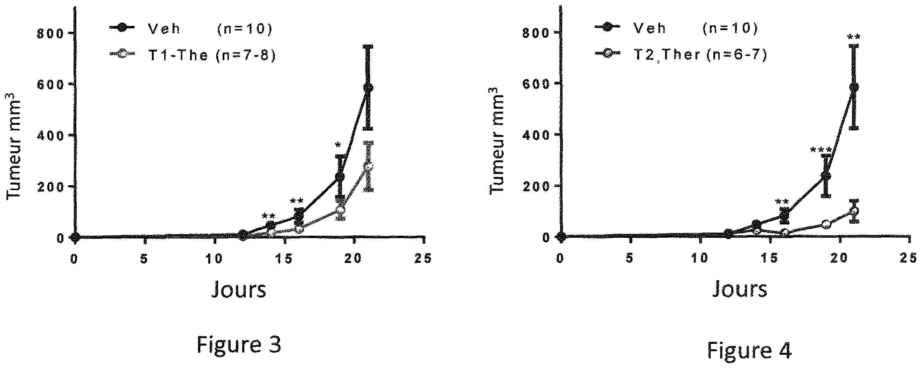

Les résultats de mesure du volume de la tumeur en mm³ au cours du temps montrent un impact favorable des deux vaccins thérapeutiques T1 et T2 par rapport aux souris témoin : voir figures 3 et 4.

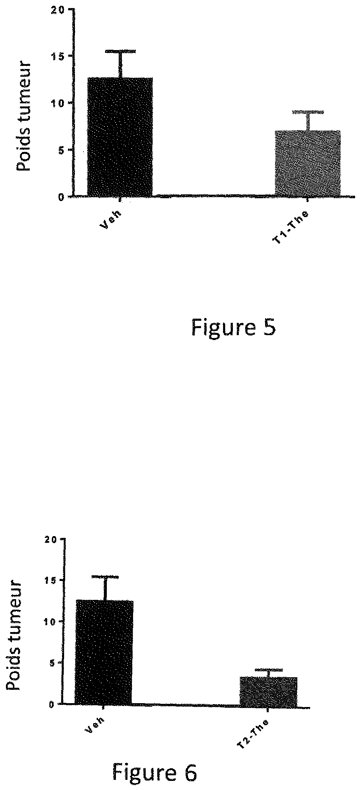

Les résultats de mesure du poids des tumeurs après résection ont également montré un impact favorable des vaccins T1 et T2 par rapport au souris témoin : voir figures 5 et 6.

## Revendications

1. Procédé de préparation d'une composition comprenant des cellules tumorales stressées et des protéines de stress immunogènes desdites cellules tumorales, comprenant les étapes suivantes :
i) cultiver des cellules tumorales dans un milieu de culture ;
ii) soumettre *in vitro* les cellules tumorales en culture à l'étape i) à un stress, dans lequel le stress est choisi dans le groupe consistant en une irradiation, un choc thermique, un choc chimique, un stress métabolique et une combinaison d'au moins deux d'entre eux,
dans lequel lesdites cellules en culture développent un mécanisme de résistance en réponse audit stress qui engendre la production des protéines de stress par lesdites cellules ;
iii) récupérer le produit de l'étape ii) comprenant des cellules tumorales stressées et des protéines de stress se trouvant en intracellulaire, libre extracellulaire, intramembranaire, et/ou en surface d'une cellule tumorale ;
iv) traiter le produit obtenu à l'étape iii) par une molécule ou un procédé capable de rendre les protéines de stress immunogènes.
dans lequel l'intervalle de temps entre la fin de l'étape ii) et le début de l'étape suivante est de plusieurs heures.

2. Procédé selon la revendication **1,** dans laquelle les cellules tumorales stressées sont rendues non-prolifératives après l'étape ii), iii) ou iv), optionnellement les cellules tumorales stressées non-prolifératives comprennent des fragments de telles cellules.

3. Procédé selon la revendication **1** ou **2,** dans lequel, à l'étape ii), les cellules tumorales sont en phase de croissance ou en phase plateau.

4. Procédé selon l'une quelconque des revendications **1** à **3,** dans lequel on introduit dans le produit issu de l'étape iii), une molécule permettant de conférer de l'immunogénicité aux protéines de stress.

5. Procédé selon l'une quelconque des revendications **1** à **4,** dans lequel le traitement à l'étape iv) comprend de soumettre le produit de l'étape iii) à une hapténisation.

6. Procédé selon la revendication **5,** dans lequel l'hapténisation comprend d'incuber le produit obtenu à l'étape iii) *in vitro* en présence d'une molécule choisie dans le groupe consistant en dinitrophényl, 2,4-dinitrofluorobenzène (DNFB), acide sulfanilique, *N*-iodoacetyl-*N*'-(5-sulfonic-naphthyl) éthylènediamine (AED), aniline, et acide p-aminobenzoique.

7. Procédé selon l'une quelconque des revendications **1** à **6,** dans lequel l'intervalle de temps entre la fin de l'étape d'application du stress ii) et le début de l'étape suivante est comprise entre environ 5 h et environ 24 h.

8. Procédé selon l'une quelconque des revendications **1** à **7,** dans lequel le stress à l'étape ii) comprend :
- une irradiation comprise entre 0,25 et 25 gray pendant une durée comprise entre environ 1 et environ 20 minutes, de préférence entre environ 1 et environ 5 minutes,
- un choc thermique entre environ 38 et environ 45°C pendant une durée comprise entre environ 20 et environ 100 minutes, de préférence entre environ 30 et environ 60 minutes,
- un choc chimique avec une ou plusieurs substances choisies parmi les alcools, par exemple l'éthanol, et les substances antitumorales utilisées en chimiothérapie, notamment du type agents alkylants, agents intercalants, par exemple le cyclophosphamide ou la doxorubicine, agents cassant pour l'ADN, par exemple le cisplatine, pendant une durée d'environ 1 à environ 48 heures,
- un stress métabolique choisi parmi une hypoxie, une modification du pH par acidification en-deçà de pH 6,5, une déficience en une substance utile ou indispensable à la croissance ou à la survie des cellules tumorales et une combinaison d'au moins deux de ceux-ci, ou
- une combinaison d'au moins deux de ces types de stress.

9. Procédé selon l'une quelconque des revendications **1** à **8,** dans lequel à l'étape ii) les cellules tumorales cultivées sont soumises à au moins deux stress différents, de préférence successivement l'un à l'autre.

10. Procédé selon la revendication **9,** dans lequel le premier stress est appliqué aux cellules tumorales cultivées, puis on observe une phase de latence, et on applique ensuite le stress suivant sur ces mêmes cellules.

11. Procédé selon l'une quelconque des revendications **1** à **10,** dans lequel le procédé est appliqué séparément à deux groupes de cellules d'une même population de cellules tumorales, avec un type de stress appliqué à un de ces groupes de cellules et un autre type de stress appliqué à l'autre de ces groupes, et les produits obtenus sont éventuellement mélangés dans la même composition.

12. Procédé selon l'une quelconque des revendications **1** à **10,** dans lequel le procédé est appliqué séparément à au moins deux populations de cellules tumorales, et les produits obtenus sont mélangés dans la même composition.

13. Procédé selon l'une quelconque des revendications **1** à **12,** dans lequel le produit obtenu est formulé avec un véhicule ou excipient pharmaceutiquement acceptable et éventuellement un adjuvant.

14. Composition comprenant des cellules tumorales stressées non-prolifératives et des protéines de stress immunogènes desdites cellules tumorales stressées, susceptible d'être obtenue par la mise en œuvre du procédé selon l'une quelconque des revendications **2** à **13,** pour son utilisation comme traitement anticancéreux, dans laquelle le stress *in vitro* auquel sont soumises les cellules tumorales est de même nature que celui qu'un patient devant être traité va subir, de préférence le stress *in vitro* auquel sont soumises les cellules tumorales comprend une irradiation lorsque la composition est destinée à un patient devant être traité par radiothérapie, et/ou le stress *in vitro* auquel sont soumises les cellules tumorales comprend un choc chimique à l'aide d'un agent de chimiothérapie lorsque la composition est destinée à un patient devant être traité par ce même agent de chimiothérapie.

15. La composition pour utilisation selon la revendication **14,** comprenant l'administration d'au moins une dose de 10⁵ à 10⁷ cellules tumorales non prolifératives au patient.

## Patentansprüche

1. Verfahren zur Herstellung einer Zusammensetzung, die gestresste Tumorzellen und immunogene Stressproteine dieser Tumorzellen umfasst, welches die folgenden Schritte umfasst:
i) Kultivieren der Tumorzellen in einem Kulturmedium;
ii) Aussetzen der Tumorzellen in Kultur in Schritt i) zu einem Stress *in vitro,* wobei der Stress ausgewählt ist aus der Gruppe bestehend aus einer Bestrahlung, einem Hitzeschock, einem chemischen Schock, einem metabolischen Stress und einer Kombination von mindestens zwei davon, wobei die Zellen in Kultur als Reaktion auf den Stress einen Resistenzmechanismus entwickeln, der die Produktion der Stressproteine durch die Zellen auslöst;
iii) Gewinnen des Produkts aus Schritt ii), das gestresste Tumorzellen und Stressproteine umfasst, die sich intrazellulär, frei extrazellulär, intramembranär und/oder an der Oberfläche einer Tumorzelle befinden;
iv) Behandeln des in Schritt iii) erhaltenen Produkts mit einem Molekül oder einem Verfahren, das in der Lage ist, die Stressproteine immunogen zu machen,
wobei die Zeitspanne zwischen dem Ende von Schritt ii) und dem Beginn des nächsten Schrittes mehrere Stunden beträgt.

2. Verfahren nach Anspruch **1,** wobei die gestressten Tumorzellen nach Schritt ii), iii) oder iv) nicht-proliferativ gemacht werden, wobei die nicht-proliferativen gestressten Tumorzellen gegebenenfalls Fragmente solcher Zellen umfassen.

3. Verfahren nach Anspruch **1** oder **2,** wobei sich die Tumorzellen in Schritt ii) in Wachstumsphase oder in Plateauphase befinden.

4. Verfahren nach einem der Ansprüche **1** bis **3,** wobei in das aus Schritt iii) hervorgehende Produkt ein Molekül eingebracht wird, das es ermöglicht, den Stressproteinen die Immunogenität zu verleihen.

5. Verfahren nach einem der Ansprüche **1** bis **4,** wobei die Behandlung in Schritt iv) das Aussetzen des Produkts aus Schritt iii) einer Haptenisierung umfasst.

6. Verfahren nach Anspruch **5,** wobei die Haptenisierung das Inkubieren des in Schritt iii) erhaltenen Produkts *in vitro* in Gegenwart eines Moleküls umfasst, ausgewählt aus der Gruppe bestehend aus Dinitrophenyl, 2,4-Dinitrofluorbenzol (DNFB), Sulfanilsäure, *N*-Iodacetyl-*N*'-(5-sulfonaphthyl)ethylendiamin (AED), Anilin und p-Aminobenzoesäure.

7. Verfahren nach einem der Ansprüche **1** bis **6,** wobei die Zeitspanne zwischen dem Ende des Schritts ii) des Anwendens des Stresses und dem Beginn des nächsten Schritts zwischen etwa 5 Stunden und etwa 24 Stunden beträgt.

8. Verfahren nach einem der Ansprüche **1** bis **7,** wobei der Stress in Schritt ii) umfasst:
- eine Bestrahlung zwischen 0,25 und 25 Gray während einer Dauer zwischen etwa 1 und etwa 20 Minuten, vorzugsweise zwischen etwa 1 und etwa 5 Minuten,
- einen Hitzeschock zwischen etwa 38 und etwa 450°C während einer Dauer zwischen etwa 20 und etwa 100 Minuten, vorzugsweise zwischen etwa 30 und etwa 60 Minuten,
- einen chemischen Schock mit einer oder mehreren Substanzen, ausgewählt aus Alkoholen, zum Beispiel Ethanol, und antitumoralen Substanzen, die in der Chemotherapie verwendet werden, insbesondere vom Typ der alkylierenden Wirkstoffe, interkalierenden Wirkstoffe, zum Beispiel Cyclophosphamid oder Doxorubicin, DNA-spaltenden Wirkstoffe, zum Beispiel Cisplatin, während einer Dauer von etwa 1 bis etwa 48 Stunden,
- einen metabolischen Stress, ausgewählt aus einer Hypoxie, einer Veränderung des pH-Werts durch Säuerung unter einen pH-Wert von 6,5, einem Mangel einer Substanz, die für das Wachstum oder das Überleben der Tumorzellen förderlich oder unverzichtbar ist, und einer Kombination von mindestens zwei davon, oder
- eine Kombination von mindestens zwei dieser Arten von Stress.

9. Verfahren nach einem der Ansprüche **1** bis **8,** wobei die kultivierten Tumorzellen in Schritt ii) mindestens zwei verschiedenen Stressen, vorzugsweise einem nach dem anderen, ausgesetzt werden.

10. Verfahren nach Anspruch **9,** wobei der erste Stress auf die kultivierten Tumorzellen angewendet wird, anschließend eine Latenzphase beobachtet wird, und danach der nächste Stress auf dieselben Zellen angewendet wird.

11. Verfahren nach einem der Ansprüche **1** bis **10,** wobei das Verfahren getrennt auf zwei Zellgruppen derselben Population von Tumorzellen angewendet wird, wobei auf eine dieser Zellgruppen eine Art von Stress angewendet wird, und auf die andere dieser Gruppen eine andere Art von Stress angewendet wird, und die erhaltenen Produkte eventuell in dieselbe Zusammensetzung gemischt werden.

12. Verfahren nach einem der Ansprüche **1** bis **10,** wobei das Verfahren getrennt auf mindestens zwei Populationen von Tumorzellen angewendet wird, und die erhaltenen Produkte in dieselbe Zusammensetzung gemischt werden.

13. Verfahren nach einem der Ansprüche **1** bis **12,** wobei das erhaltene Produkt mit einem pharmazeutisch verträglichen Träger oder Hilfsstoff und eventuell einem Adjuvans formuliert wird.

14. Zusammensetzung, die nicht-proliferative gestresste Tumorzellen und immunogene Stressproteine der gestressten Tumorzellen umfasst, die durch Umsetzung des Verfahrens nach einem der Ansprüche **2** bis **13** erhalten werden kann, zu deren Verwendung als Antikrebsbehandlung, wobei der *in vitro*-Stress, dem die Tumorzellen ausgesetzt werden, von derselben Art ist wie der, dem ein zu behandelnder Patient unterzogen werden soll, wobei vorzugsweise der *in vitro-*Stress, dem die Tumorzellen ausgesetzt werden, eine Bestrahlung umfasst, wenn die Zusammensetzung für einen Patienten bestimmt ist, der mittels Strahlentherapie behandelt werden soll, und/oder der *in vitro*-Stress, dem die Tumorzellen ausgesetzt werden, einen chemischen Schock mithilfe eines Chemotherapie-Wirkstoffs umfasst, wenn die Zusammensetzung für einen Patienten bestimmt ist, der mit demselben Chemotherapie-Wirkstoff behandelt werden soll.

15. Zusammensetzung zur Verwendung nach Anspruch **14,** welche die Verabreichung mindestens einer Dosis von 10⁵ bis 10⁷ nicht-proliferativen Tumorzellen an den Patienten umfasst.

## Claims

1. A method of preparing a composition comprising stressed tumor cells and immunogenic stress proteins of said tumor cells, comprising the following steps:
i) cultivating tumor cells in a culture medium;
ii) subjecting the cultured tumor cells from step i) to a stress *in vitro,* wherein the stress is selected from the group consisting of an irradiation, a heat shock, a chemical shock, a metabolic stress and a combination of at least two of them,
wherein said cultured cells develop a resistance mechanism in response to said stress which causes the production of the stress proteins by said cells;
iii) recovering the product of step ii) comprising stressed tumor cells and stress proteins found intracellularly, extracellularly free, intramembranously, and/or at the surface of a tumor cell;
iv) treating the product obtained at step iii) with a molecule or a process capable of rendering the stress proteins immunogenic,
wherein the time interval between the end of step ii) and the beginning of the next step is of several hours.

2. The method according to claim **1,** wherein the stressed tumor cells are rendered non-proliferative after step ii), iii) or iv), optionally the non-proliferative stressed tumor cells comprise fragments of such cells.

3. The method according to claim **1** or **2,** wherein, at step ii), the tumor cells are in growth phase or in plateau phase.

4. The method according to any one of claims **1** to **3,** wherein a molecule capable of rendering the stress proteins immunogenic is introduced in the product of step iii).

5. The method according to any one of claims **1** to **4,** wherein the treatment at step iv) comprises subjecting the product of step iii) to haptenization.

6. The method according to claim **5,** wherein the haptenization comprises incubating the product obtained at step iii) *in vitro* in presence of a molecule selected from the group consisting of dinitrophenyl, 2,4-dinitrofluorobenzene (DNFB), sulfanilic acid, *N*-iodoacetyl-*N*'-(5-sulfonic-naphthyl) ethylenediamine (AED), aniline, and *p*-aminobenzoic acid.

7. The method according to any one of claims **1** to **6,** wherein the time interval between the end of stress application at step ii) and the beginning of the next step is between about 5 h and about 24 h.

8. The method according to any one of claims **1** to **7,** wherein the stress at step ii) comprises:
- an irradiation of between 0.25 and 25 gray for a period of between about 1 and about 20 minutes, preferably of between about 1 and about 5 minutes,
- a heat shock between about 38 and about 45°C for a period of between about 20 and about 100 minutes, preferably of between about 30 and about 60 minutes,
- a chemical shock with one or several substances chosen among alcohols, for example ethanol, and antitumor substances used in chemotherapy, in particular alkylating agents, intercalating agents, for example cyclophosphamide or doxorubicin, DNA breaking agents, for example cisplatin, for a period of about 1 to about 48 hours,
- a metabolic stress chosen among hypoxia, pH alteration by acidification below pH 6.5, deficiency of a useful or essential substance for tumor cell growth or survival and a combination of at least two of them, or
- a combination of at least two of these types of stress.

9. The method according to any one of claims **1** to **8,** wherein at step ii), the cultured tumor cells are subjected to at least two different stresses, preferably successively to each other.

10. The method according to claim **9,** wherein the first stress is applied to cultured tumor cells, then a latency phase is observed, and then the next stress is applied to these same cells.

11. The method according to any one of claims **1** to **10,** wherein the method is applied separately to two groups of cells of a same tumor cell population, with one type of stress being applied to one of these groups of cells and another type of stress being applied to the other of these groups, and the products obtained are optionally mixed within the same composition.

12. The method according to any one of claims **1** to **10,** wherein the method is applied separately to at least two populations of tumor cells, and the products obtained are mixed within the same composition.

13. The method according to any one of claims **1** to **12,** wherein the product obtained is formulated with a pharmaceutically acceptable vehicle or excipient and optionally an adjuvant.

14. A composition comprising non-proliferative stressed tumor cells and immunogenic stress proteins of said stressed tumor cells, obtainable by the implementation of the method according to any one of claims **2** to **13,** for use as anticancer treatment, wherein the stress the tumor cells are subjected to *in vitro* is of the same nature as the one a patient in need of treatment will undergo, preferably the stress the tumor cells are subjected to *in vitro* comprises an irradiation when the composition is intended for a patient to be treated by radiotherapy, and/or the stress the tumor cells are subjected to *in vitro* comprises a chemical shock with a chemotherapeutic agent when the composition is intended for a patient to be treated with the same chemotherapeutic agent.

15. The composition for use according to claim 14, comprising the administration of at least one dose of 10⁵ to 10⁷ non-proliferative tumor cells to the patient.
